# EUROPEAN PATENT APPLICATION

(11) **EP 2 796 531 A1**
(43) Date of publication of application: **29.10.2014**
(21) Application number: 12860443.6
(22) Date of filing: 20.12.2012
(51) Int. Cl.: C09K 11/08, C01G 29/00, C09K 11/00, C09K 11/67

(54) **AGENT FOR IMPARTING FLUORESCENCE TO CERAMIC**

(30) Priority: 22.12.2011 JP 2011281707; 18.12.2012 JP 2012275364
(71) Applicant: GC Corporation, Tokyo 113-0033 (JP)
(72) Inventor: MASHIO Go, Tokyo 174-8585 (JP); KARIYA Syuji, Tokyo 174-8585 (JP)
(74) Representative: Müller-Boré & Partner Patentanwälte PartG mbB
(86) International application number: PCT/JP2012/083009
(87) International publication number: WO 2013/094669

(57) **Abstract**

Provided is a fluorescence imparting agent for ceramics that does not require the use of an acidic solution during production. The fluorescence imparting agent for ceramics is a water solution containing 5 to 90 % by weight of glycerin and 0.01 to 10 % by weight of one or more bismuth compound selected from bismuth bromide (BiBr3), bismuth bromate (Bi(Br03)3), bismuth chloride (BiCl3), bismuth chlorate (Bi(ClO3)3), bismuth fluoride (BiF3), bismuth pentafluoride (BiF5), bismuth hydroxide (Bi(OH)3), bismuth iodide (BiI3), bismuth iodate (Bi(IO3)3), bismuth nitrate (Bi(N03)3), bismuth subnitrate (Bi5O(OH)9(NO3)4), bismuth phosphate (BiPO4), and bismuth sulfate (Bi2(SO4)3).

## Description

The present invention relates to a fluorescence imparting agent for ceramics which imparts fluorescence to ceramics.

Ceramic materials have been widely used. For example, in the case of manufacturing a dental prosthesis which is to be installed into an oral cavity, a block made of a zirconia material including a small amount of stabilizing material such as a zirconium oxide and a yttrium oxide is employed, and the block is cut out into a desired shape according to a CAD/CAM technology. A zirconia block is formed as a calcined substance by applying a calcination process to a molded body and is thereafter provided for a manufacturing facility such as a dental laboratory in a state of a sintered material to which a calcination process is further applied. Then, a technical worker in the processing facility creates a frame from the zirconia block according to a model prepared by a dentist, and a prosthetic appliance is manufactured by building up a porcelain on the frame.

However, the zirconia (zirconium oxide) is lower in its light permeability and fluorescence in comparison with a natural tooth, and appears a different color tone. Accordingly, there has been a problem in the light of an aesthetic property. Therefore, fluorescence which is close to the fluorescence of the natural tooth has been imparted by applying the calcination process to a molded body obtained by mixing a powder including elements Pr, Sm, Eu, Ga, Gd, Tm, Nd, Dy, Tb and Er serving as a fluorescence component to the zirconia including a small amount of stabilizing material so as to form the calcined substance, and thereafter applying the calcination process (refer, for example, to patent document 1).

On the other hand, as a simpler method, there has been a method of imparting appropriate fluorescence to the zirconia by applying a calcined substance of the molded zirconia to a solution (a fluorescence imparting agent) including a bismuth compound such as bismuth chloride, bismuth hydroxide or bismuth nitrate by way of dipping or applying the solution, and thereafter applying the calcination process. However, in the prior art, since it is necessary to use a lot of acid solution (particularly, hydrochloric acid, sulfuric acid, nitric acid and mixed acid of them which are frequently used in industry) for dissolving the bismuth compound in the case of manufacturing the solution, great danger has been caused, and productivity has been deteriorated.
Patent Document 1: Japanese Unexamined Patent Publication No. 2010-222466

Accordingly, an object of the present invention is to provide a fluorescence imparting agent for ceramics which does not need to use an acid solution in the case of manufacturing the fluorescence imparting agent for ceramics.

The inventors of the present invention have devoted themselves to make a study for solving the problem mentioned above. As a result, the inventors find that a mixed liquid obtained by mixing a particular bismuth compound and a water solution including glycerin becomes an acid solution capable of dissolving the bismuth compound, and therefore it is possible to manufacture the fluorescence imparting agent for ceramics which is equivalent to the conventional one without using any acid solution at a time of manufacturing, and the inventors have then completed the present invention.

In other words, the present invention is a fluorescence imparting agent for ceramics comprising a water solution which includes 5 to 90 % by weight of glycerin, and 0.01 to 10 % by weight of one or more bismuth compound which is or are selected from bismuth bromide (BiBr3), bismuth bromate (Bi(BrO3)3), bismuth chloride (BiCl3), bismuth chlorate (Bi(ClO3)3), bismuth fluoride (BiF3), bismuth pentafluoride (BiF5), bismuth hydroxide (Bi(OH)3), bismuth iodide (BiI3), bismuth iodate (Bi(IO3)3), bismuth nitrate (Bi(N03)3), bismuth subnitrate (Bi5O(OH)9(NO3)4), bismuth phosphate (BiP04), and bismuth sulfate (Bi2(SO4)3).

It is preferable that the fluorescence imparting agent for ceramics according to the present invention further includes 5 to 80 % by weight of one or more than two kinds of alcohol which is or are selected from diglycerin, polyglycerin having less than 20 glycerin units, erythritol, pentaerythritol, dipentaerythritol, arabitol, ribitol, xylitol, sorbitol, mannitol, galactitol, inositol, quercitol, 1,2-propanediol, dipropylene glycol, pentanediol, 13-butanediol, 1,2-ethanediol, 3-oxapentane-1,5-diol, triethylene glycol, methanol, ethanol, 2-propanol, 2-methyl-2-propanol, 1-pentanol, 2-pentanol, 3-pentanol, 2-methyl-1-butanol, 3-methyl-1-butanol, 2-methyl-2-butanol, 3-methyl-2-butanol, and 2,2-dimethyl-1-propanol.

Further, it is preferable that the fluorescence imparting agent for ceramics according to the present invention further includes 0.01 to 10 % by weight of one or more thulium compound which is or are selected from thulium boride (TmB6), thulium bromide (TmBr3), thulium chloride (TmCl3), thulium fluoride (TmF3), thulium hydride (TmH3), thulium iodide (TmI3), thulium nitrate (Tm(NO3)3), thulium oxide (Tm2O3), and thulium hydroxide (Tm(OH)3).

The fluorescence imparting agent for ceramics according to the present invention is an excellent fluorescence imparting agent for ceramics which does not need to use any acid solution at a time of manufacturing.

Bismuth compound to be used in the present invention is one or more powdery compound which is or are selected from bismuth bromide (BiBr3), bismuth bromate (Bi(Br03)3), bismuth chloride (BiCl3), bismuth chlorate (Bi(ClO3)3), bismuth fluoride (BiF3), bismuth pentafluoride (BiF5), bismuth hydroxide (Bi(OH)3), bismuth iodide (BiI3), bismuth iodate (Bi(IO3)3), bismuth nitrate (Bi(NO3)3), bismuth subnitrate (Bi5O(OH)9(NO3)4), bismuth phosphate (BiPO4), and bismuth sulfate (Bi2(SO4)3). The bismuth compounds become acid by being mixed with the water solution including glycerin and facilitate mixing of the bismuth compounds. Accordingly, it is only necessary to use the water solution including glycerin and the powdery bismuth compound at a time of manufacturing, and it is not necessary to use acid solution as solution before being mixed.

It is necessary that powdery bismuth compound is included at 0.01 to 10 % by weight in the fluorescence imparting agent for ceramics. In the case that the amount of the powdery bismuth compound is less than 0.01 % by weight, the effect of the fluorescence imparting agent for ceramics is hard to be obtained, and it tends to be hard in the light of solubility to blend the powdery bismuth compound beyond 10 % by weight, and a fluorescence intensity tends to be rather lowered. The amount of the powdery bismuth compound is preferably from 0.1 to 7 % by weight, and further preferably from 0.2 t 1 % by weight.

Glycerin has an effect of easily dissolving the particular bismuth compound by being used as the water solution. A blending amount of the glycerin in the fluorescence imparting agent for ceramics is between 5 and 90 % by weight, is preferably between 15 and 80 % by weight, and is further preferably between 20 and 55 % by weight. The effect of dissolving the bismuth compound tends to be lowered in the other ranges than the above.

The fluorescence imparting agent for ceramics according to the present invention may be further mixed with an alcohol in the light of the solubility of the bismuth compound and an operability in the case of applying the solution to the block. The alcohol is constituted by one or more alcohol which is or are selected from diglycerin, polyglycerin having less than 20 glycerin units, erythritol, pentaerythritol, dipentaerythritol, arabitol, ribitol, xylitol, sorbitol, mannitol, galactitol, inositol, quercitol, 1,2-propanediol, dipropylene glycol, pentanediol, 1,3-butanediol, 1,2-ethanediol, 3-oxapentane-1,5-diol, triethylene glycol, methanol, ethanol, 2-propanol, 2-methyl-2-propanol, 1-pentanol, 2-pentanol, 3-pentanol, 2-methyl-1-butanol, 3-methyl-1-butanol, 2-methyl-2-butanol, 3-methyl-2-butanol, and 2,2-dimethyl-1-propanol. The blending amount of the alcohols is from 5 to 80 % by weight in the fluorescence imparting agent for ceramics, is preferably from 6 to 40 % by weight, and is further preferably from 8 to 25 % by weight.

Thulium compound may be further included in the fluorescence imparting agent for ceramics according to the present invention. The thulium compound imparts fluorescence thereto by interacting with a small amount of stabilizing material, for example, yttrium oxide which is included in the block of the zirconia. It is possible to impart higher fluorescence by being used together with the particular bismuth compound mentioned above on the basis of this action.

The thulium compound is constituted by one or more thulium compound which is or are selected from thulium boride (TmB6), thulium bromide (TmBr3), thulium chloride (TmCl3), thulium fluoride (TmF3), thulium hydride (TmH3), thulium iodide (TmI3), thulium nitrate (Tm(N03)3), thulium oxide (Tm2O3), and thulium hydroxide (Tm(OH)3). It is preferable that the thulium compound is blended in the fluorescence imparting agent for ceramics in a range from 0.01 to 10 % by weight. In the case that the amount is less than 0.01 % by weight, the effect is hard to be obtained, and it is not preferable in the light of a cost in relation to the effect to blend beyond 10 % by weight. The amount is more preferably from 0.1 to 3 % by weight, and is further preferably from 0.2 to 1 % by weight.

### Embodiment

52.3 g of glycerin (produced by Wako Pure Chemical Industries, Ltd.) was added to 40.7 g of water, and was mixed and agitated for thirty minutes. The water solution of glycerin was mixed with 7 g of bismuth nitrate (produced by Wako Pure Chemical Industries, Ltd.) and was further agitated for a hundred and twenty minutes so as to obtain a fluorescence imparting agent for ceramics (an embodiment 1). According to the same method, there were prepared fluorescence imparting agents for ceramics (embodiments 2 to 7) according to the present invention and fluorescence imparting agents for ceramics (comparative examples 1 and 2) which were manufactured by a conventional manufacturing method using the acid solution as comparative examples, on the basis of compositions described in Table 1. In the present embodiments and the comparative examples, the fluorescence imparting agents for ceramics are prepared by g units of numerical values described in Table 1, however, in the actual manufacturing according to the prior art, it was necessary to carry at least several tens of liters of concentrated hydrochloric acid (concentration of hydrogen chloride: 37 % by weight), for example, for manufacturing 100 kg in 1 lot, and handle 10 kg of concentrated hydrochloric acid (in the case of the composition of the comparative example 1) at a time of mixing. Accordingly, a great danger was caused and an efficiency was deteriorated.

### <Evaluation of Fluorescence>

The evaluation of the fluorescence was carried out by measuring a sintered material obtained by dipping a calcined substance which was formed as a disc shape having a diameter of 15 mm and a thickness of 0.6 mm from the zirconia processed under the same condition, and to which a calcination process was applied, in the fluorescence imparting agent for ceramics according to each of the embodiments and each of the comparative examples for two minutes and drying, and thereafter applying a calcination process thereto, by means of a microplate reader (brand name SpectraMax M2, produced by Molecular Devices Japan), and comparing obtained intensities of fluorescence (relative fluorescence units). Results are shown in Table 1.

**Table 1**

| | | Embodiments | | | | | | | Comparative examples | |
|---|---|---|---|---|---|---|---|---|---|---|
| | | 1 | 2 | 3 | 4 | 5 | 6 | 7 | 1 | 2 |
| bismuth compound | bismuth nitrate | 7 | 5.2 | 0.2 | - | 0.1 | - | - | - | - |
| | bismuth hydroxide | - | - | - | 0.3 | - | - | 0.2 | - | |
| | bismuth chloride | - | - | - | - | 0.2 | 0.2 | - | 2 | 0.2 |
| glycerin | | 52.3 | 49.7 | 30.6 | 49.7 | 29.3 | 23.8 | 26.6 | - | - |
| water | | 40.7 | 25.1 | 49 | 49 | 60 | 69 | 51.6 | 65 | 69 |
| alcohol | 1,2-propanediol | - | 20 | - | - | 10.2 | - | 8 | 22 | - |
| | triethylene glycol | - | - | 20 | - | - | 6.8 | 13.3 | - | 25.6 |
| thulium compound | thulium chloride | - | - | - | 1 | 0.2 | 0.1 | - | 1 | 0.2 |
| | thulium nitrate | - | | 0.2 | - | - | 0.1 | 0.3 | - | - |
| acid | concentrated hydrochloric acid (37 % by weight) | - | - | - | - | - | - | - | 10 | 5 |
| fluorescence intensity (relative fluorescence unit) | | 270000 | 290000 | 300000 | 300000 | 300000 | 300000 | 300000 | 300000 | 300000 |

As shown in Table 1, in the fluorescence imparting agents for ceramics according to the present invention, it is possible to obtain the fluorescence imparting agent for ceramics having the same fluorescence intensity as the conventional one without using any acid solution at a time of manufacturing the fluorescence imparting agent for ceramics.

## Claims

1. A fluorescence imparting agent for ceramics comprising:
a water solution which includes 5 to 90 % by weight of glycerin, and 0.01 to 10 % by weight of one or more bismuth compound selected from the group consisting of bismuth bromide (BiBr3), bismuth bromate (Bi(Br03)3), bismuth chloride (BiCl3), bismuth chlorate (Bi(ClO3)3), bismuth fluoride (BiF3), bismuth pentafluoride (BiF5), bismuth hydroxide (Bi(OH)3), bismuth iodide (BiI3), bismuth iodate (Bi(IO3)3), bismuth nitrate (Bi(N03)3), bismuth subnitrate (Bi5O(OH)9(NO3)4), bismuth phosphate (BiPO4), and bismuth sulfate (Bi2(SO4)3).

2. The fluorescence imparting agent for ceramics according to claim 1, further comprising 5 to 80 % by weight of one or more alcohol selected from the group consisting of diglycerin, polyglycerin having less than 20 glycerin units, erythritol, pentaerythritol, dipentaerythritol, arabitol, ribitol, xylitol, sorbitol, mannitol, galactitol, inositol, quercitol, 1,2-propanediol, dipropylene glycol, pentanediol, 1,3-butanediol, 1,2-ethanediol, 3-oxapentane-1,5-diol, triethylene glycol, methanol, ethanol, 2-propanol, 2-methyl-2-propanol, 1-pentanol, 2-pentanol, 3-pentanol, 2-methyl-1-butanol, 3-methyl-1-butanol, 2-methyl-2-butanol, 3-methyl-2-butanol, and 2,2-dimethyl-1-propanol.

3. The fluorescence imparting agent for ceramics according to claim 1 or 2, further comprising 0.01 to 10 % by weight of thulium compound.

4. The fluorescence imparting agent for ceramics according to claim 3, wherein the thulium compound is one or more thulium compound selected from the group consisting of thulium boride (TmB6), thulium bromide (TmBr3), thulium chloride (TmCl3), thulium fluoride (TmF3), thulium hydride (TmH3), thulium iodide (TmI3), thulium nitrate (Tm(N03)3), thulium oxide (Tm2O3), and thulium hydroxide (Tm(OH)3).
